# EUROPÄISCHE PATENTSCHRIFT

(11) **EP 1 905 466 B1**
(45) Veröffentlichungstag und Bekanntmachung des Hinweises auf die Patenterteilung: **31.05.2017**
(21) Anmeldenummer: 06020436.9
(22) Anmeldetag: 28.09.2006
(51) Int. Cl.: A61M 5/14

(54) **BESTIMMUNG EINES INJEKTIONSBEREICHS IN EINER HETEROGENEN KÖRPERSTRUKTUR**
IDENTIFICATION OF AN INFUSION REGION IN THE HETEROGENEOUS TISSUE
INDENTIFICATION D'UNE RÉGION D'INFUSION DANS UN TISSUE HÉTÉROGÈNE

(43) Veröffentlichungstag der Anmeldung: 02.04.2008
(73) Patentinhaber: Brainlab AG, 81829 München (DE)
(72) Erfinder: Rodriguez Ponce, Maria Immaculada, 81669 München (DE)
(74) Vertreter: Schwabe - Sandmair - Marx

(56) Entgegenhaltungen:
- EP-A- 1 376 443
- EP-A- 1 396 233
- EP-A1- 1 316 324
- EP-A1- 1 398 641
- EP-A1- 1 479 403
- WO-A2-01/85230
- WO-A2-2006/085288

## Beschreibung

Die vorliegende Erfindung betrifft die Bestimmung eines geeigneten Infusions und/oder Injektionsbereichs zur Infusion und/oder Injektion eines medizinischen Wirkstoffes in eine anisotrope, insbesondere heterogene, Körperstruktur. Biologisches Gewebe, wie zum Beispiel Gehirn oder Leber sind im allgemeinen heterogene Körperstrukturen, die Eigenschaften aufweisen, die dazu führen, dass sich eine injizierte Substanz nicht isotrop um die Injektionsstelle herum ausbreitet. Somit kann sich der Zielbereich, an dem der medizinische Wirkstoff wirkt, deutlich von dem Injektionspunkt unterscheiden, an dem der medizinische Wirkstoff injiziert wird. Dieser Effekt wird durch die Heterogenität und die unterschiedlichen physikalischen und physiologischen Eigenschaften der betroffenen Körperstrukturen bewirkt. Diese Unterschiede können bereits bei einer gesunden Körperstruktur (z.B. anatomische und/oder biologische und/oder physiologische Eigenschaften der Körperstrukturen) gegeben sein, sie können aber auch erst durch Erkrankungen (z.B. Tumor oder Blutungen) erzeugt oder verstärkt werden. Beispielsweise ist die Vaskularität eines Tumors sehr komplex. Die vom Tumor neu gebildeten Zellen und Gefäße können sich sich deutlich in ihren Eigenschaften von den Zellen und Gefäßen gesunden Gewebes unterscheiden. Beispielsweise haben Blutgefäße eines Hirntumors, insbesondere von Gliablastomen, keine funktionell wirksame Blut-Hirn-Schranke. So ist bei Gliablastomen die Zahl der endothelialen Vesikel deutlich erhöht und es finden sich Lücken in der Endothelschicht. Allgemein zeigt sich eine deutliche Veränderung der Extrazellularmatrix pathologischer Gefäße in Gliablastomen (das gesunde Gehirn umfasst Aushöhlungen und Falten, die die Verteilung der Flüssigkeit beeinflussen).

Die oben genannten Besonderheiten anisotroper Körperstrukturen führten in der Vergangenzeit zu Fehlschlägen bei gezielter lokaler Chemotherapie von Gliablastomen im Gehirn. Während bei Laborversuchen deutliche Erfolge erzielt werden konnten, konnten diese am Menschen nicht wiederholt werden. Dies wird wesentlich darauf zurückgeführt, dass der medizinische Wirkstoff (im Fall von Gliablastomen insbesondere ein Chemotherapeutikum) aufgrund der Heterogenität des Gehirns sich nicht oder nicht ausreichend in dem gewünschten Zielbereich ausgebreitet hat, sondern durch die Heterogenität des Gewebes fehlgeleitet wurde. Dies führt insbesondere zu Problemen bei der so genannten druckgetriebenen Wirkstoffabgabe CED ("Convection-Enhanced Delivery") zur Behandlung von Gehirntumoren.

Aus EP 1 398 641 A1 ist eine Verteilungsbestimmung zur Infusionsplanung bekannt. Offenbart ist ein Verfahren zur Identifizierung von vorteilhaften bzw. nicht vorteilhaften Infusionsbereichen im Gewebe, bei dem funktionelle und/oder strukturelle Anatomiedaten erfasst werden, und bei dem computergestützt eine Auswertung der Anatomiedaten im Hinblick auf die darin enthaltenen Verteilungsinformationen, insbesondere Richtungs- und/oder Geschwindigkeitsinformationen durchgeführt wird sowie ein Verfahren zur Planungsunterstützung bzw. zur Navigationsunterstützung für die Einbringung eines Infusionsfluids. Sie betrifft ferner eine Vorrichtung zur Planungsunterstützung für die Einbringung eines Infusionsfluids, insbesondere in Gehirnbereiche.

Aus EP 1 316 324 A1 ist eine Vorrichtung zur Verabreichung einer Substanz bekannt. Offenbart ist ein Verfahren zur Planung einer Infusion, wobei Patientendaten erfasst werden und die durchzuführende Infusion unter Verwendung der erfassten Patientendaten geplant wird; sowie auf ein Computerprogramm, welches in den Speicher eines Computers geladen werden kann und Softwarecodeabschnitte umfasst, mit welchen das Verfahren ausgeführt wird, wenn das Programm auf einem Computer läuft; sowie auf eine Vorrichtung zur Planung einer Infusion mit einem Patientendatenerfassungssystem und einem Computersystem zur Durchführung der Planung basierend auf den erfassten Patientendaten; auf ein Verfahren zum Durchführen einer Infusion, wobei eine Planung der Infusion und anschließend die Infusion durchgeführt wird; und eine Vorrichtung zur Durchführung einer Infusion mit einer Verifikationsvorrichtung zum Vergleichen von geplanten Infusionsdaten mit tatsächlichen Infusionsdaten.

Aus EP 1 376 443 A1 ist ein Verfahren und eine Vorrichtung zur Bestimmung von Migrationswegen von Zellen bekannt. Offenbart ist ein Verfahren zum Bestimmen von Migrationswegen von Zellen, wobei Daten eines Körpers durch ein bildgebendes Verfahren erfasst werden, aus den erfassten Daten für Migrationswege relevante anatomische Informationen gewonnen werden, und für den Körper Migrationswege berechnet warden sowie auf eine Vorrichtung zur Durchführung eines bildgebenden Verfahrens und einer Auswerteeinheit zur individuellen Ermittlung von Migrationswegen in einem Körper basierend auf den von der Vorrichtung zur Verfügung gestellten Daten.

Aus WO 01/85230 A2 ist ein Verfahren und ein Apparat bekannt, das die Injektion von Medikamenten betrifft. Dabei wird die Bewegung des im Gehirn injizierten Medikaments durch ein gleichförmiges Feld statischer Konstanten modelliert, die den Transport durch Fluidbewegung und Diffusion innerhalb des Fluids bestimmen.

Aus WO 2006/085288 A2 ist ein Verfahren zum Bestimmen eines Injektionspunkts bekannt. Das Medikament wird dabei in ein Gefäß eines Zielbereichs injiziert.

EP 1 396 233 A1 betrifft ein Positionssystem für ein neurologisches Verfahren im Gehirn.

DE 60 2004 009 243 T2 offenbart ein Verfahren zu der Stoffwechsel betreffenden Führung von eingeleitetem zellulärem Material. Insbesondere werden im Rahmen jener Erfindung Modelle für das Zellverhalten genutzt und ein Strömungsgeschwindigkeitsfeld für ein sich bewegendes Fluid definiert. Das Parameterfeld und das Strömungsgeschwindigkeitsfeld werden geschätzt, um die Entwicklung der Konzentration eines infundierten Fluid vorherzusagen. Es wird ferner bestimmt, ob die erwarteten Ergebnisse für die Konzentration und Zellaktivität akzeptabel sind oder sich Veränderungen in den angenommenen Werten aufgrund der Dynamik ergeben haben. Es wird die Bewegung des Fluids simuliert, wobei ein Richtungswert berechnet wird und die Konzentration im Zielgewebegebiet optimiert wird. Die erwartetete Konzentration kann dann mit einer tatsächlich gemessenen Konzentration verglichen werden, um das Parameterfeld entsprechend anzupassen.

Aufgabe der Erfindung ist es, ein Verfahren, insbesondere ein Planungsverfahren, und eine Vorrichtung zur Planung bereitzustellen, das bzw. die es erlaubt, einen Infusions- und/oder DE 60 2004 009 243 T2 offenbart ein Verfahren zu der Stoffwechsel betreffenden Führung von eingeleitetem zellulärem Material. Insbesondere werden im Rahmen jener Erfindung Modelle für das Zellverhalten genutzt und ein Strömungsgeschwindigkeitsfeld für ein sich bewegendes Fluid definiert. Das Parameterfeld und das Strömungsgeschwindigkeitsfeld werden geschätzt, um die Entwicklung der Konzentration eines infundierten Fluid vorherzusagen. Es wird ferner bestimmt, ob die erwarteten Ergebnisse für die Konzentration und Zellaktivität akzeptabel sind oder sich Veränderungen in den angenommenen Werten aufgrund der Dynamik ergeben haben. Es wird die Bewegung des Fluids simuliert, wobei ein Richtungswert berechnet wird und die Konzentration im Zielgewebegebiet optimiert wird. Die erwartetete Konzentration kann dann mit einer tatsächlich gemessenen Konzentration verglichen werden, um das Parameterfeld entsprechend anzupassen.

Injektionsbereich zu bestimmen, der so optimiert ist, dass ein gewünschtes Zielvolumen mit erhöhter Wahrscheinlichkeit erreicht werden kann.

Vorstehende Aufgabe wird durch die Gegenstände der unabhängigen Ansprüche gelöst. Vorteilhafte Weiterbildungen gehen aus den Unteransprüchen hervor.

Vorteilhaft wird ein optimaler Injektionsbereich zur Infusion oder Injektion eines medizinischen Wirkstoffs bestimmt, um in einem gewünschten Zielbereich die optimale Verteilung zu erzielen. Dies insbesondere auch dann, wenn der gewünschte Zielbereich nicht direkt zugänglich ist, sondern nur indirekt durch Injektion oder Infusion an anderer Stelle erreicht werden kann. Wichtige anisotrope Parameter und Kenngrößen sind die räumliche Verteilung der hydraulischen Permeabilität, der kapilaren Permeabilität, der hydraulischen Eigenschaften (Flüssigkeitsleitfähigkeit), Parameter, die die Zellmatrix, insbesondere die räumliche Veränderung des extrazellulären Raums beschreiben, der Vaskularität des Tumors und der Tumormatrixeigenschaften, wie dies weiter unten noch ausgeführt wird. Vorteilhaft erlaubt die Erfindung, die beste Lage (Stelle) für die Infusion oder Injektion vorzuschlagen, und wie viele Injektionsbereiche notwendig sind, um den gewünschten Zielbereich zu erreichen und abzudecken.

Vorteilhaft können durch die Erfindung Infusions- und/oder Injektionsbereiche vorgeschlagen werden, ohne dass, wenn dies gewünscht ist, zuvor ein Katheter platziert wird und ein Injektionsergebnis gemessen wird, um aus den hieraus gewonnenen Erfahrungen schrittweise die Lage der Injektionsbereiche zu optimieren.

Das erfindungsgemäße Verfahren wird vorzugsweise eingesetzt, um mindestens einen Infusions- und/oder Injektionsbereich zu bestimmen. Der Infusions- und/oder Injektionsbereich soll so bestimmt werden, dass in einem gewünschten Zielbereich der injizierte medizinische Wirkstoff wirkt, insbesondere mit einer gewünschten Konzentration wirkt.

Das Verfahren beruht darauf, Eigenschaften der heterogenen Körperstruktur zu verwenden, um den mindestens einen Infusions- und/oder Injektionsbereich zu bestimmen. Bei diesen Eigenschaften handelt es sich vorzugsweise um Eigenschaften, die die räumliche Verteilung des medizinischen Wirkstoffes, insbesondere die Verteilung der Konzentration des medizinischen Wirkstoffes in der heterogenen Körperstruktur beeinflussen, also desjenigen medizinischen Wirkstoffes, der zur Injektion in dem mindestens einen vorgegebenen Infusions- und/oder Injektionsbereich der heterogenen Körperstruktur vorgesehen ist. "Heterogen" bedeutet hierin insbesondere eine räumliche Variation der vorgenannten Eigenschaften oder Parameter. Sie sind demnach insbesondere an verschiedenen Orten der Körperstruktur unterschiedlich und nicht homogen ausgebildet. Bei den Eigenschaften handelt es sich insbesondere um Eigenschaften, die den Wirkstofftransportund/oder Wirkstoffverlust beeinflussen.

Vorzugsweise wird erfindungsgemäß der mindestens eine Infusions- und/oder Injektionsbereich in Abhängigkeit von den vorgenannten Eigenschaften der heterogenen Körperstruktur und der Lage des gewünschten mindestens einen Zielbereichs berechnet.

Vorteilhaft werden die vorgenannten Eigenschaften der heterogenen Körperstruktur, die die räumliche Verteilung des medizinischen Wirkstoffes in der heterogenen Körperstruktur beeinflussen, durch eine den Transport des medizinischen Wirkstoffes beeinflussenden Verbundenheit ("connectivity") von Segmenten der heterogenen Körperstruktur beschrieben. Die Verbundenheit kann ein Grad und/oder ein Maß der Verbindung der Segmente sein, kann aber auch vereinfacht binär beschrieben werden, d.h. keine Verbindung oder eine Verbindung. Die Verbundenheit beschreibt insbesondere den Umfang des Wirkstofftransportes von einem Segment zu einem verbundenen Segment, insbesondere benachbarten Segment. Die Segmente können als Elemente einer Matrix beschrieben werden. Sie können allerdings auch unterschiedliche Größe und/oder Gestalt haben. Der Begriff "Wirkstofftransport" umfasst hierin nicht nur aktive Transportvorgänge, z.B. hervorgerufen durch Strömungen und/oder Druckgradienten, sondern z.B. auch passive Transportvorgänge, Migration, Diffusion, Osmose, Perfusion etc. Also alle Vorgänge, die zu einer Veränderung der räumlichen Lage des Wirkstoffes führen.

Für die medizinische Wirkung des Wirkstoffes ist die räumliche Verteilung der Wirkstoffkonzentration von Bedeutung. Diese wird nicht nur durch den Wirkstofftransport beeinflusst sondern auch durch Wirkstoffverluste. Wirkstoffverluste können ebenfalls durch Wirkstofftransportvorgänge bewirkt werden, falls der Wirkstoff beispielsweise in Bereiche außerhalb der betrachteten anisotropen, insbesondere heterogenen, Körperstruktur oder in für die medizinische Wirkung nicht interessante unerwünschte Bereiche transportiert. Eine wesentliche Ursache für Wirkstoffverluste ist die Permeabilität von Teilen der anisotropen Körperstruktur, also beispielsweise von Zell- und/oder Gefäßwänden. Eine Verteilung der Wirkstoffkonzentration in der anisotropen Körperstruktur und insbesondere eine Ausbreitung des Wirkstoffes in der heterogenen Körperstruktur wird bevorzugt bestimmt, indem sowohl der Wirkstofftransport als auch der Wirkstoffverlust berücksichtigt wird. Bei einer bevorzugten Ausführungsform wird eine Unterscheidung zwischen Wirkstofftransport und Wirkstoffverlust dadurch getroffen, dass der Wirkstofftransport einen Transport des Wirkstoffes von einem Bereich (Segment) zu einem anderen Bereich (Segment) der betrachteten heterogenen Körperstruktur bewirkt, während der Wirkstoffverlust eine Wirkstoffverringerung in einem betrachteten Bereich (Segment) der heterogenen Körperstruktur bewirkt, ohne dass dies zu einer Erhöhung der Wirkstoffmenge oder der Wirkstoffkonzentration in einem anderen Bereich der für die Berechnung betrachteten heterogenen Körperstruktur führt. Vorzugsweise werden insbesondere Bereiche mit hoher kapillarer Permeabilität und somit einem hohen Risiko von Verlusten nicht als geeigneter Infusions- und/oder Injektionsbereich angesehen und somit vorzugsweise von dem erfindungsgemäßen Verfahren als Infusions- und/oder Injektionsbereich ausgeschlossen.

Vorteilhaft wird die Verbundenheit und/oder der Wirkstofftransport und/oder der Wirkstoffverlust durch die hydraulischen Eigenschaften der Segmente, insbesondere die kapillaren Permeabilität ("capillar permeability") und/oder die hydraulische Leitfähigkeit ("hydraulic conductivity") beschrieben (auch als "hydraulic permeability" bekannt). Im Folgenden wird die kapillare Permeabilität verkürzt als "Permeabilität" bezeichnet. Insbesondere wird zur Beschreibung der Verbundenheit auf die Anordnung der Elemente, insbesondere auf ihre Nachbarschaftsbeziehungen zueinander, Bezug genommen. Werte, die hydraulischen Eigenschaften der Segmente beschreiben, werden vorzugsweise auf ihre Ähnlichkeit und/oder ihre Differenz hin untersucht, um die Verbundenheit ("connectivity") der Elemente zu bestimmen. Haben beispielsweise zwei benachbarte Segmente eine hohe hydraulische Leitfähigkeit, so ist eine gute Verbundenheit (hoher Grad der Verbindung) der Segmente gegeben und es kann von einem guten Wirkstofftransport ausgegangen werden. Haben beispielsweise benachbarte Segmente eine niedrige Permeabilität, so eignen sich diese Segmente gut zum Aufbau einer hohen Wirkstoffkonzentration, da der Wirkstoff nicht verloren geht. Die Verbundenheit kann sowohl für die hydraulische Leitfähigkeit als auch für die kapillare Permeabilität definiert werden. Die Verbundenheit, die sich auf die kapillare Permeabilität bezieht, gibt im Wesentlichen Informationen über den Umfang des Verlustes. Je größer die Verbundenheit ist, umso größer wird der Verlust sein. Die Verbundenheit, die sich auf die hydraulische Leitfähigkeit bezieht, gibt Informationen über die räumliche Verteilung des Fluids aber auch indirekt Informationen über die Konzentration des Wirkstoffes.

Der Wirkstofftransport von einem Bereich zu einem anderen Bereich kann beispielsweise physikalisch durch das pro Zeiteinheit transportierte Wirkstoffgewicht und/oder Wirkstoffvolumen und/oder durch Beschreibung der Konzentrationsänderungen erfolgen. Entsprechend kann physikalisch der Wirkstoffverlust durch die Änderung des Wirkstoffvolumens und/oder der Wirkstoffkonzentration in einem Bereich, insbesondere pro Zeiteinheit beschrieben werden.

Auch die Größe und/oder Gestalt und/oder Rigidität bzw. Steifigkeit ("rigidity") der Segmente und/oder die Anzahl der Nachbarschaftsbeziehungen (Verbindungen zu benachbarten Segmenten) wird vorzugsweise berücksichtigt, um die Verbundenheit zu bestimmen.

Auch kann eine Wahrscheinlichkeitsberechnung zur Bestimmung der Verbundenheit durchgeführt werden. Diese gründet auf der Wahrscheinlichkeit eines Wirkstofftransportes von einem Segment zu einem weiteren Segment. Hierbei wird vorzugsweise auf die Theorie der Perkolation zurückgegriffen, wie man dies insbesondere aus der Geologie kennt. In der Geologie beschreibt die Perkolation die Wanderung kleinster, wanderungsfähiger Bodenteilchen, so genannter Perkolate im Porenraum eines Bodens. Diese Theorie kann auf anisotrope Körperstrukturen angewendet werden, um die Wanderung bzw. den Transport des Wirkstoffes in der heterogenen Körperstruktur zu beschreiben. Dabei können insbesondere Perkolationspfade des Wirkstoffes in der heterogenen Körperstruktur berechnet werden. Gemäß der Perkolationstheorie kann man zum Beispiel bei der Theorie der Kantenperkolation (bond percolation) die Wahrscheinlichkeit einer Verbindung eines Segments zu einem Nachbarsegment berechnen und somit Perkolationspfade bestimmen. Insbesondere wird ein richtungsabhängiger Verbindungsgrad (Verbundenheit) einer Zelle zu einer Nachbarzelle dabei bestimmt. Dies kann beispielsweise mit einem Vektorfeld oder einem Tensorfeld beschrieben werden, wobei jedem Segment ein Vektor oder Tensor zugeordnet ist, der Richtung und Größe der Verbindung beschreibt.

Bei den medizinischen Wirkstoffen kann es sich um Molekülgruppen, z.B. hochspezifische Toxine mit spezifischen Bindungskörpern (z.B. Antigene oder Antikörper), die hochselektiv an Gliablastome ankoppeln, um Fluide, Liposome, Mikrosphären, Compounds, Viren, virale Vektoren, Zellen, zelluläre Bestandteile, Erbgutmoleküle, Nanopartikel oder andere therapeutisch wirksame Substanzen handeln, die insbesondere von Flüssigkeiten transportiert werden.

Vorteilhafterweise werden die berechneten Perkolations-Pfade, die bestimmten Verbundenheiten zwischen den Segmenten oder entlang eines Pfades, ein Wahrscheinlichkeitsplan, der eine Wahrscheinlichkeit für die Distribution des Wirkstoffes in einem jeden Segment bei vorgegebenen Injektionsbereichen darstellt sowie vorgeschlagene Injektionsbereiche visualisiert und beispielsweise an einem Monitor dargestellt. Die vorgenannten Visualisierungen sind beispielhaft. Einzelne können herausgegriffen werden und mit anderen Visualisierungen kombiniert werden.

Vorteilhaft wird erfindungsgemäß ein zu erwartendes Verhältnis und/oder Beziehung der Konzentration und/oder der Menge des zur Infusion und/oder Injektion vorgesehenen medizinischen Wirkstoffes in dem mindestens einem Wirkbereich zu der injizierten Konzentration und/oder Menge berechnet. Dies erlaubt die Bestimmung des mindestens einen Injektionsbereichs zu optimieren. Vorzugsweise wird ein Infusions- und/oder Injektionsbereich ausgewählt, für den sich bei minimaler Injektionsmenge eine maximale Wirkstoffsdistribution im gewünschten Zielbereich ergibt. Vorteilhaft wird das zuvor genannte zu erwartende Verhältnis (und/oder Beziehung) und/oder die (absolute und/oder relative) Konzentration und/oder die (absolute und/oder relative) Menge des zur Injektion vorgesehenen medizinischen Wirkstoffes zumindest in Abhängigkeit mindestens einer Eigenschaft der Injektionsbedingungen des medizinischen Wirkstoffes und/oder mindestens einer Eigenschaft des medizinischen Wirkstoffes berechnet.

Vorteilhaft wird die zu erwartende, von den Zellen im Wirkbereich absorbierte Menge und/oder absorbierte Menge pro Zeiteinheit berechnet. Dies wird vorzugsweise basierend auf der gerechneten Konzentration im Wirkbereich und Absorptionseigenschaften der Zellen im Wirkbereich berechnet. Dabei wird insbesondere auf Gleichungen zurückgegriffen, die chemische Gleichgewichtsreaktionen zwischen Wirkstoff und Zelle beschreiben.

Gemäß einer weiteren Ausbildung des erfindungsgemäßen Verfahrens wird vorzugsweise eine angestrebte Konzentration im Zielbereich, die Sollwirkkonzentration genannt wird, vorgegeben. Diese Sollwirkkonzentration stellt eine gewünschte Konzentration des zur Infusion und/oder Injektion vorgesehenen medizinischen Wirkstoffes in dem Zielbereich dar. Basierend hierauf wird dann durch das erfindungsgemäße Verfahren mindestens eine anzustrebende Injektionsbedingung zur Injektion und/oder Infusion des medizinischen Wirkstoffes bestimmt. Injektionsbedingungen sind beispielsweise die Menge des injizierten Wirkstoffes pro Zeiteinheit, die Wirkstoffkonzentration, die Injektionsrichtung, der Injektionsort, die Trajektorie der Injektionsvorrichtung (z.B. Katheter oder Nadel), der Injektionsdruck, der Injektionsstromfluss, die Injektionsdauer. Die Injektionsbedingung wird vorzugsweise basierend auf der bestimmten Lage des mindestens einen Infusions und/oder Injektionsbereichs berechnet, so dass die Sollinjektionsbedingungen zu der gewünschten Sollwertkonzentration im Zielbereich führen.

Die vorgenannten Eigenschaften der anisotropen, insbesondere heterogenen Körperstruktur, die, wie zuvor ausgeführt wurde, beispielsweise durch die Permeabilität und hydraulische Leitfähigkeit und Verbundenheit beschrieben werden kann, kann auch zumindest eine der folgenden Eigenschaften umfassen oder durch diese dargestellt werden:

Eigenschaften, die den Transport des medizinischen Wirkstoffes, insbesondere von mittels Flüssigkeit transportfähigen Wirkstoffstrukturen in der heterogenen Körperstruktur beeinflussen. Die Beeinflussung kann insbesondere auch durch Körperflüssigkeit-Transportvorgängen erfolgen, also beispielsweise von Strömungen, insbesondere Blutströmungen entlang von Gefäßen.

Ein weiteres Beispiel für derartige Eigenschaften ist der Verlauf und die Lage von Senken für den medizinischen Wirkstoff der heterogenen Körperstruktur. Beispiele sind hier poröse Blutgefäße, wie sie insbesondere in Tumoren vorkommen, die zu einem insbesondere unerwünschten verstärkten Abfluss des medizinischen Wirkstoffes führen, bevor dieser den gewünschten Wirkbereich erreichen kann.

Eine weitere beispielhafte Eigenschaft ist die Absorption des medizinischen Wirkstoffes in der anisotropen, insbesondere heterogenen Körperstruktur insbesondere durch gesunde Zellen, wohingegen insbesondere die Absorption durch krankhafte Zellen gewünscht ist.

Noch ein weiteres Beispiel für die Eigenschaften ist die Diffusion des medizinischen Wirkstoffes, der den Wirkbereich erreicht hat und diesen durch Diffusion wieder verlässt. Weitere Beispiele sind wie folgt:

Die Permeabilität von in der anisotropen, insbesondere heterogenen, Körperstruktur verlaufenden, zum Wirkstofftransport geeigneten Gefäßen. Die Transportleistung der Gefäße für den Wirkstoff, die beispielsweise durch die Fließgeschwindigkeit der Flüssigkeit in den Gefäßen, den Gefäßdurchmesser und den Fluss der Flüssigkeit in dem Gefäß beschrieben werden kann. Die durchschnittliche Aufenthaltsdauer des Wirkstoffes (z.B. beschreibbar durch eine Konzentrationszerfallszeit in dem Gefäß oder in den Zellen). Die hydraulische und/oder Wirkstoff-Leitfähigkeit von Zellen zu benachbarten Zellen oder von Gefäßen zu benachbarten Gefäßen, allgemein von einem Segment zu einem benachbarten Segment. Die Perkolationseigenschaften der heterogenen Körperstruktur. Die vorgesehene Lage des Patienten, und die dadurch bedingten Gravitationskräfte, die die Perkolation des Wirkstoffes beeinflussen können.

Die vorgenannten Gefäße und Zellen stellen Beispiele von Teilen der anisotropen, insbesondere heterogenen, Körperstruktur dar. Weitere Beispiele sind Nervenstränge und Sulci. Soweit hierin von Teilen der Körperstrukturen die Rede ist, können die Teile sich in ihren biologischen Eigenschaften und insbesondere Wirkstoff-Transporteigenschaften und/oder Wirkstoffverlusteigenschaften unterscheiden. So haben Tumorzellen andere Wirkstofftransporteigenschaften als Nervenstränge. Insbesondere kann die anisotrope Körperstruktur zur erfindungsgemäßen Bestimmung des Injektionsbereichs räumlich in Segmente aufgeteilt werden. Die Segmente können so gestaltet werden, dass sie jeweils einem Teil der Körperstruktur zugeordnet sind, der eine spezifische Wirkstofftransporteigenschaft hat. Somit können Daten für jedes Segment der Körperstruktur vorgegeben werden, die die Wirkstofftransporteigenschaft dieses Segments und/oder einen Wirkstoffverlust in diesem Segment beschreiben. Basierend auf diesen Daten kann dann die Wirkstoffausbreitung und/oder der Wirkstoffverlust in der Körperstruktur, insbesondere räumlich aufgelöst, z.B. segmentweise berechnet werden.

Die für die Bestimmung der räumlichen Verteilung der Verbundenheit und/oder des Wirkstofftransportes und/oder des Wirkstoffverlustes verwendeten Segmente können kleiner sein als die vorgenannten (charakteristischen) Teile der Körperstruktur (z.B. Gefäße oder Zellen, Nervenstränge). Sie können aber auch größer sein, so dass ihnen ein mittleres Maß der Verbundenheit und/oder des Wirkstofftransportes zu benachbarten Segmenten und/oder des Wirkstoffverlustes dann zugeordnet werden kann.

Beispiele von Injektionseigenschaften sind:
- die zur Injektion vorgesehene Menge des medizinischen Wirkstoffes, z.B. integriert über die gesamte Injektionsdauer;
- die vorgesehene Injektionsdauer
- die zur Injektion vorgesehene Konzentration des medizinischen Wirkstoffes;
- die pro Zeiteinheit vorgesehene Menge der Injektion des medizinischen Wirkstoffes;
- die vorgesehen Injektionsrichtung des medizinischen Wirkstoffes. Diese kann beispielsweise auch durch ein Verbiegen des Katheters beeinflusst werden;
- die vorgesehene Flussgeschwindigkeit der Injektion;
- der vorgesehene Druck der Injektion;
- die vorgesehene Gestaltung des zur Injektion vorgesehenen Injektionsgerätes, insbesondere die Größe und/oder Gestalt der Austrittsöffnung im Injektionsgerät, das zur Injektion des medizinischen Wirkstoffes vorgesehen ist;
- die vorgesehene Lage des Injektionsgerätes in der Körperstruktur, insbesondere die Position und/oder Orientierung der Austrittsöffnung und/oder die vorgesehene Lage und/oder Größe des vom Injektionsgerät eingenommen Volumens in der Körperstruktur;
- die Lage und/oder Gestalt der Oberfläche des vom Injektionsgerät in der Körperstruktur;
- der vorgesehene Verlauf eines durch die Einfuhr des Injektionsgeräts erzeugten Einführkanals, insbesondere Lage, Größe und Form des Einführkanals.

Beispiele für Wirkstoffeigenschaften, die zur Bestimmung des optimalen Injektionsbereichs verwendet werden können, sind folgende:
- Größe und/oder Gestalt der Wirkstoffstrukturen;
- Transportfähigkeit (Diffusion, Strömung, Migration) der Wirkstoffstrukturen in Flüssigkeiten, insbesondere in der Körperflüssigkeit und/oder der Injektionsflüssigkeit;
- Viskosität der Injektionsflüssigkeit;
- chemische und/oder biologische und/oder physiologische Eigenschaften des medizinischen Wirkstoffes;
- metabolische Eigenschaften des Wiorkstoffes
- Durchdringungseigenschaften des medizinischen Wirkstoffes durch Barrieren, wie zum Beispiel Zellwände und/oder Blut-Hirn-Schranke.

Die Erfindung betrifft weiter die Planung des mindestens einen Infusions- und/oder Injektionsbereichs, um einen optimalen Injektionsbereich für eine Injektion oder Infusion des medizinischen Wirkstoffes erzielen zu können, wobei bei diesem Planungsverfahren das vorbeschriebene Verfahren zur Bestimmung des mindestens einen Injektionsbereichs verwendet wird. Die Erfindung kann bevorzugt als ein Computerprogramm ausgeführt sein, das das vorgenannte Planungsverfahren einsetzt, wobei, wenn das Computerprogramm auf einen Computer geladen wird, der Computer dazu veranlasst wird, das Planungsverfahren durchzuführen.

Weiter betrifft die Erfindung eine Vorrichtung, die ein Berechnungsmittel (z.B. eine Datenverarbeitungseinrichtung) umfasst, um das vorgenannte Verfahren zur Bestimmung des mindestens einen Infusions- und/oder Injektionsbereichs basierend auf der Eingabe mindestens eines gewünschten Zielbereichs durchzuführen. Vorgenannte Vorrichtung kann weiter ein Injektionsinstrument zum Injizieren des medizinischen Wirkstoffes umfassen. Die Vorrichtung kann alternativ oder zusätzlich eine Analyseeinheit (Überwachungseinheit), beispielsweise ein Röntgengerät zur Bestimmung der Lage des Injektionsbereichs umfassen, falls das Injektionsinstrument in die anisotrope Körperstruktur eingeführt wurde, um so zu überprüfen, ob der gewünschte Injektionsbereich erreicht wurde. Weiter kann die vorgenannte Vorrichtung Diagnoseeinrichtungen umfassen, die die Bestimmung der Eigenschaften der Körperstruktur erlauben. Weiter kann die vorgenannte Datenverarbeitungseinheit eine Datenbank umfassen, die Eigenschaften von Teilen der Körperstruktur beschreibt, die die Verteilung des Wirkstoffes in der Körperstruktur beeinflussen, beispielsweise die Permeabilität bzw. Starrheit von biologisch sich unterscheidenden Teilen der Körperstruktur, wie z.B. von Tumorzellen und Tumorgefäßen und von gesunden Zellen und gesunden Gefäßen sowie von Sulci und/oder Nervenbahnen, sowie die hydraulische Leitfähigkeit derartiger Teile.

Daten, die die Eigenschaften der heterogenen Körperstruktur beschreiben, die die räumliche Verteilung des medizinischen Wirkstoffes in der anisotropen, insbesondere heterogenen, Körperstruktur beeinflussen werden vorzugsweise durch Diagnoseeinheiten ermittelt. Insbesondere wird vorzugsweise auf das so genannte MR-DTI (diffusion tensor imaging) zurückgegriffen. Auch können andere Magnetresonanzverfahren und/oder Ultraschallverfahren und/oder Kontrastverfahren verwendet werden, um diese Eigenschaften zu bestimmen. Beispiele stellen dar Magnetresonanztomographie (MRT), Magnetresonanzangiographie (MRA), funktionelle MRT (fMRI), Bildgebung des zellularen Blutflusses (ASL), Diffusionsbildgebung (DWI), Perfusionsbildgebung (PWI), MR-Spektroskopie (MRS, SVS, CSI) sowie die bereits erwähnte Diffusions-Tensor-Bildgebung (DTI), die bevorzugt verwendet wird. Letztere erlaubt insbesondere Auskunft über die räumliche Verteilung und Mobilität von Wassermolekülen und liefert Hinweis auf die morphologische Integrität der weißen und grauen Substanz des Gehirns. Sie erlaubt eine Differenzierung der Molekülbewegungen in mehrere Raumrichtungen und somit insbesondere die oben im Zusammenhang mit der Perkolationstheorie erwähnten Tensor- und/oder Vektormatrizen, die die Verbundenheit von Segmenten beschreiben können. Die Wirkstoffdiffusion, insbesondere die Moleküldiffusion wird durch unterschiedliche Barrieren, wie zum Beispiel Zellwände oder membranöse Strukturen innerhalb der Zellen eingeschränkt. Auch krankhafte Veränderungen, wie ischämische, entzündliche, demyelinisierende Gewebeveränderungen des Gehirns beeinflussen ebenfalls das Diffusionsverhalten von Molekülen und insbesondere Wassermolekülen und Wirkstoffmolekülen. Auch ist beispielsweise entlang der Nervenfasern die Beweglichkeit deutlich größer als senkrecht zum Faserverlauf. So ergibt sich beispielsweise durch die Richtungsabhängigkeit (Anisotropie) der Molekülbewegungen eine Information über den Verlauf und insbesondere die Integrität cerebraler Faserverbindungen. Auch dies kann zur Bestimmung der Verbundenheit benachbarter Segmente, insbesondere die Richtung der Verbundenheit und das Maß der Verbundenheit in Abhängigkeit von der Richtung verwendet werden.

Bei der folgenden Beschreibung einer Ausführungsform werden weitere Merkmale der Erfindung offenbart, wobei als Abwandlung beschriebene Merkmale miteinander kombiniert werden können.

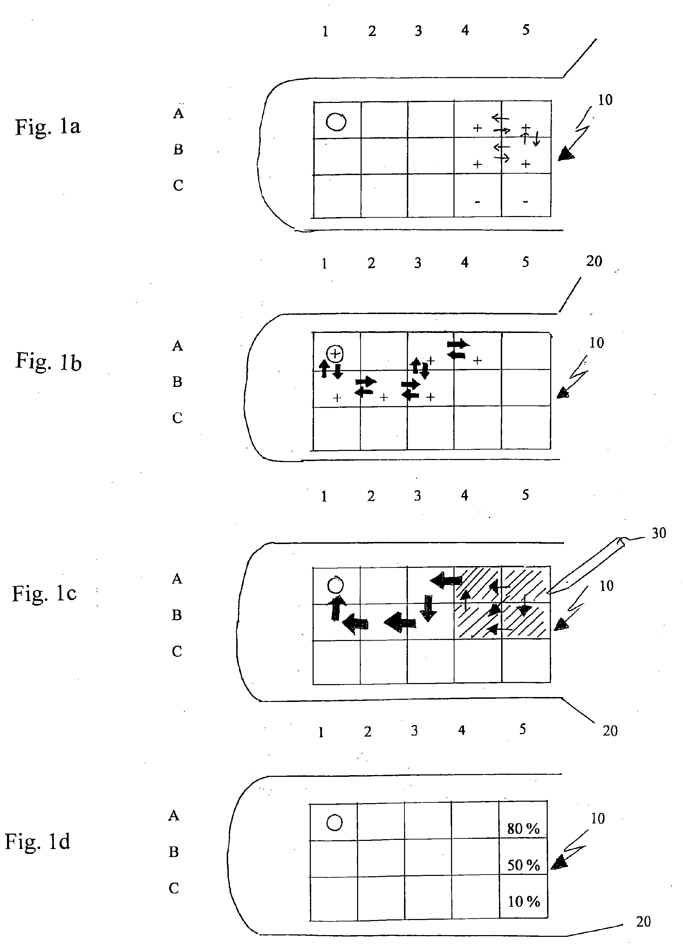

Figur 1a zeigt eine Matrix von Segmenten, wobei den Segmenten Permeabilitätswerte zugeordnet sind;
Figur 1b zeigt eine Matrix mit Segmenten, wobei den Segmenten ein Leitfähigkeitswert zugeordnet ist;
Figur 1c zeigt einen Perkolationspfad von einem Injektionsbereich zu einem Wirkbereich;
Figur 1d zeigt eine Wahrscheinlichkeitsmatrix.

Bei dem im Folgenden beschriebenen Verfahren zur Bestimmung eines Infusions- und/oder Injektionsbereichs, genauer bei dieser Ausführungsform eines zur Infusion geeigneten Segments in der anisotropen, insbesondere heterogenen, Körperstruktur werden Werte über die (hydraulische) Permeabilität der Segmente mit Werten über die hydraulische Leitfähigkeit der Segmente kombiniert, um einen Perkolationspfad von einem möglichen Injektionssegment zu dem gewünschten Zielbereich, genauer hier Zielsegment zu erzielen. Weitere Eigenschaften der Körperstruktur können natürlich zur Bestimmung des Pfades und/oder des geeigneten Injektionssegments herangezogen werden. Auch kann nur entweder die hydraulische Permeabilität oder die hydraulische Leitfähigkeit zur Berechnung herangezogen werden.

Die Segmentmatrix 10 umfasst in dem Beispiel 15 Elemente. Die Reihen der Matrix sind mit A, B und C bezeichnet, die Spalten mit 1, 2, 3, 4 und 5, um so auf die einzelnen Segmente bei der folgenden Beschreibung Bezug nehmen zu können.

Das gewünschte Zielsegment ist mit einem Kreis bezeichnet. Es ist das Segment A1. Es können natürlich auch mehrere Wirksegmente gewünscht sein, die benachbart oder separiert sein können. Mit 20 ist schematisch eine Barriere bezeichnet, die die Form eines seitwärts liegenden "U" hat. Sie soll andeuten, dass ein Durchdringen dieser Barriere zum Erreichen eines Injektionssegment mit einem Injektionsinstrument nicht möglich ist oder ein Einführpfad für das Injektionsinstrument (z.B. Katheter, Spritze, Nadel), das die Barriere 20 oder allgemein den Bereich 20 durchdringt, ist aus medizinischen Gründen unerwünscht. Ein für den Einführpfad unerwünschter Bereich 20 kann beispielsweise mit erhöhtem unerwünschten so genannten Rückfluss (backflow) des Wirkstoffes entlang des eingestochenen Injektionsinstruments und somit unerwünschten Wirkstoffverlust begründet sein oder die Verfolgung des Verlaufs des Einführpfades beispielsweise mit bildgebenden Diagnosegeräten (Röntgen) kann erschwert sein oder die unerwünschte Verletzung von Körperstrukturen kann befürchtet werden.

Aufgrund der Barriere 20 wird somit angenommen, dass es bei der in Figur 1 gezeigten Situation als mögliche Injektionssegmente nur die Segmente A5, B5 und C5 gibt. Das gewünschte Zielsegment ist jedoch A1. Durch das erfindungsgemäße Verfahren soll nun bestimmt werden, ob eines der Segmente A5, B5 oder C5 für eine Injektion geeignet ist, um im Zielsegment A1 eine Wirkung zu erzielen. Falls nein, kann von einer Injektion abgesehen werden. Falls ja, soll durch das erfindungsgemäße Verfahren bestimmt werden, welches der für die Injektion zur Verfügung stehenden Segmente das geeignetste ist.

Für eine Infusion und/oder Injektion geeignete Segmente sind Segmente mit niedrigem Wirkstoffverlust, da sich dort eine Konzentration des Wirkstoffes aufbauen kann, die dann durch druckgetriebene Ausbreitung und/oder Diffusion weiter verbreitet wird. Segmente mit hohem Wirkstoffverlust können als Senken für die Wirkstoffkonzentration aufgefasst werden. Ein Wirkstoffverlust kann durch Transport des Wirkstoffes (z.B. Diffusion, passiver Transport, druckgetriebner Transport, Ionentransport, Transport aufgrund von Membranpotential, Osmose, aktiver Transport) beispielsweise durch eine Membran einer Zelle hervorgerufen werden. Dies bedeutet, dass insbesondere eine niedrige Permeabilität der Membran für Wirkstofftransportvorgänge, den Aufbau einer hohen Wirkstoffkonzentration begünstigt. Die Permeabilität von Gefäßen ist beispielsweise durch Perfusionmessungen mit Magnetresonanz-, Ultraschall oder CT-Techniken messbar und kann somit dem erfindungsgemäßen Verfahren als Information zur weiteren Berechnung zur Verfügung gestellt werden. Segmente, für die eine niedrige Permeabilität erkannt wurde, sind somit günstig, da in ihnen nur ein geringer Wirkstoffverlust auftritt. Segmente hoher Permeabilität stellen eine Senke für den Wirkstoff dar, da er über diese Segmente verloren geht. Beispiele für derartige Zellen sind Tumorblutgefäße im Gehirn mit einer hochpermeablen Gefäßwand. Segmente mit niedrigem Wirkstoffverlust (niedriger Permeabilität) haben somit vorteilhafte Injektionseigenschaften und sind in Figur 1a mit + bezeichnet. Segmente mit hohem Wirkstoffverlust (hoher Permeabilität), in denen ein hoher Wirkstoffverlust zu befürchten ist, sind in Figur 1a mit - bezeichnet.

In Figur 1a sind durch einfache Pfeile die mögliche Ausbreitung der Wirkstoffkonzentration angedeutet. Ein Maß für den Wirkstoffverlust kann beispielsweise eine mittlere Verweildauer der Wirkstoffkonzentration in einem Segment sein.

Aus Figur 1a ist ersichtlich, dass von den für die Injektion zur Verfügung stehenden Segmenten A5, B5 und C5 die Segmente A5 und B5 geeignet wären. Besonders geeignet erscheint das Segment A5, das sich bei Injektion in A5 der Wirkstoff durch langsame Diffusion in die benachbarten Segmente A4 und B4 und B5 ausbreiten kann, ohne dass der Wirkstoff aufgrund hoher Permeabilität verloren geht. Es ist somit möglich in den Zellen A4, A5, B4 und B5 eine hohe Wirkstoffkonzentration aufzubauen, da keine Verluste zu höheren Konzentrationen führen. Es kann jedoch vorkommen, dass eine hohe Verbundenheit und somit eine größere räumliche Verteilung zu einer niederen Konzentration führt. Dies hängt vom Transporttyp ab. Falls der Transportprozess diffusionsgetrieben ist, kann dies zu niedrigeren Konzentrationen führen. Falls er druckgetrieben ist, bedingt normalerweise eine hohe Verbundenheit keine niedrigere Konzentration. Insbesondere die in Figur 1 gezeigten Segmente sind jeweils eine Mischung aus Blutzellen, Zellen und Matrixgewebe (der Raum zwischen den Zellen). Dies ist insbesondere bedingt durch die räumliche Auflösung der Abbildungstechniken und somit das Datenmaterial. Bei einer höheren Auflösung können einzelne Segmente auch einzelnen Blutzellen zugeordnet werden. Das Fluid wird durch die Gewebematrix ("tissue matrix") wandern und die kapillare Permeabilität bezieht sich insbesondere auf die Gefäße oder Kapillaren. Ein Segment hoher/niedriger Permeabilität bedeutet somit, dass in diesem Segment ein Risiko hoher/niedriger Verluste besteht. Ein Segment mit hoher/niedriger Permeabilität bedeutet ein hohes/niedriges Risiko an Fluidverlusten. Andererseits liegt der Einfluss der hydraulischen Leitfähigkeit darin, dass unabhängig von den Gefäßen an diesem Ort der Transport verstärkt oder vermindert werden kann, weil die Matrix heterogen ist. Segmente mit hohem Wirkstoffverlust (einer hohen Permeabilität) sind in Figur 1a mit "-" gekennzeichnet. Somit ist das Segment 5C nicht zur Injektion des Wirkstoffes geeignet, da dort der Wirkstoff rasch die Zelle verlässt und somit keine Wirkstoffkonzentration aufgebaut werden kann. Von den übrigen Zellen A1 bis A3, B1 bis B3 und C1 bis C3 wird davon ausgegangen, dass dort eine mittlerer Wirkstoffverlust gegeben ist, der für eine Ausbreitung des Wirkstoffes mittels Diffusion von den Zellen A4, A5, B4 und B5 nach A1 nicht günstig ist oder dass für diese Bereiche keine Informationen über die Wirkstoffverluste vorliegen.

Neben dem Mechanismus der Ausbreitung des Wirkstoffes durch Diffusion gibt es auch andere Transportmechanismen, wie z.B. eine Ausbreitung mittels hydraulischer Leitfähigkeit. Dies wird weiter unten im Zusammenhang mit Figur 1b diskutiert und kann genutzt werden, den Wirkstoff zu dem gewünschten Wirksegment zu transportieren.

Figur 1b zeigt eine Matrix, die die hydraulische Leitfähigkeit betrifft. In den mit "+" gekennzeichneten Segmenten ist eine hohe hydraulische Leitfähigkeit gegeben. Beispielsweise kann ein Gefäß oder ein Nervenstrang entlang A4, A3, B3, B2, B1 und A1 verlaufen.

Entlang gesunder Gefäße oder Nervenzellen ist eine hohe hydraulische Leitfähigkeit gegeben, die für den Wirkstofftransport günstig ist. In Figur 1b ist der mögliche Transport des Wirkstoffes aufgrund der hydraulischen Leitfähigkeit durch dicke Pfeile angezeigt. Insbesondere ist eine Verbundenheit zwischen den Segmenten A3 und A4 gegeben, so dass eine Wirkstoffdistribution im Segment A4 über die Segmente A4, A3, B3, B2, B1 nach A1 möglich ist. Wie zuvor ausgeführt, ist die Verbindung zwischen dem Segment B3 und B4 aufgrund der Eigenschaften von Gefäßwänden quer zur Transportrichtung gering, so dass von einer Wirkstoffausbreitung von dem Segment B4 nach B3 nicht ausgegangen werden kann.

Den in Figur 1a und 1b gezeigten Pfeilen können Werte oder Wahrscheinlichkeiten zugeordnet werden, die den Grad und die Richtung der Verbundenheit benachbarter Segmente beschreiben. Insbesondere können die Werte der Figur 1a und Figur 1b miteinander kombiniert werden, um einen Transportpfad (hierin auch Perkolationspfad genannt) des Wirkstoffes von einem Injektionssegment zu einem Wirksegment bestimmen zu können. Insbesondere kann basierend auf den Werten oder Wahrscheinlichkeiten die Effektivität des Wirkstofftransportes berechnet werden, so dass die einzuspritzende Wirkstoffmenge aus der gewünschten Wirkstoffkonzentration im Zielsegment berechnet werden kann.

Figur 1c zeigt eine Matrix, die die Wirkstoffausbreitung entlang der Segmente beschreibt. Sie wird hierin auch Perkolationsmatrix genannt. Die dünnen Pfeile bezeichnen die Ausbreitung des Wirkstoffes in den schraffierten Segmenten A4, A5, B4 und B5. In diesen Segmenten baut sich eine hohe Wirkstoffkonzentration ausgehend vom Segment A5 aus. Die Diffusion von dem Segment A5 in die benachbarten Segmente A4, B4 und B5 ist durch einen dünnen Pfeil gekennzeichnet. Von dem Segment A4 wird dann der Wirkstoff durch die hydraulische Leitfähigkeit entlang der Segmente A4, A3, B3, B2, B1 und A1 abtransportiert. Durch den Abtransport des Wirkstoffes aus dem Segment A4 ergibt sich hier eine Erniedrigung des Wirkstoffes, die durch Diffusion aus dem Segment A5 aber auch aus dem Segment B4 ausgeglichen werden kann, was durch kleine Pfeile angedeutet ist.

Das gewünschte Wirksegment kann somit durch Injektion des Wirkstoffes in dem Segment A5 erzielt werden.

Figur 1d zeigt eine Wahrscheinlichkeitsmatrix, bei der aufgrund der Daten über die Permeabilität und hydraulische Leitfähigkeit für die möglichen Injektionssegmente A5, B5 und C5 eine Wahrscheinlichkeit angegeben wird, dass bei Injektion bei dem jeweiligen Segment eine gewünschte Wirkstoffkonzentration im Zielsegment A1 erzielt werden kann. Dabei können natürlich auch Randbedingungen berücksichtigt werden, wie Wirkstoffeigenschaften und Injektionseigenschaften wie beispielsweise die maximal einspritzbare Wirkstoffmenge, insbesondere auch pro Zeiteinheit.

Durch das erfindungsgemäße Verfahren ist es möglich Infusions- und/oder Injektionsbereiche zu bestimmen, die sich in Hinsicht auf die erreichbaren Zielbereiche unterscheiden. Dies ist insbesondere dann von Vorteil, wenn eine Injektion im Zielbereich nicht möglich ist oder dort bei direkter Injektion aus falscher Richtung zu einem hohen Wirkstoffverlust führen würde. Insbesondere kann auch bestimmt werden, ob eine Vielzahl von Zielsegmenten durch Injektion in einen Injektionsbereich oder mehrere Injektionsbereiche erreicht werden kann, um so zum Beispiel möglichst viele oder alle Zielzellen (z.B. Tumorzellen) mit dem medizinischen Wirkstoff zu erreichen. Herkömmlicherweise werden mehrere Infusionsbereiche, beispielsweise vier, vorgesehen, die um den gewünschten Zielbereich herumliegen. Berücksichtigt man jedoch nicht die Anisotropie der Körperstruktur, so können manche oder sogar alle Injektionsbereiche sich als ungeeignet erweisen, so dass der gewünschte Zielbereich von dem verwendeten Wirkstoff nicht erreicht wird oder eine gewünschte Wirkstoffkonzentration im Zielbereich nicht erreicht werden kann. Durch die vorliegende Erfindung kann dies verhindert werden. Schließlich kann man durch das erfindungsgemäße Verfahren auch planen, welche Bereiche erreicht werden sollen. Beispielsweise kann nur ein Bereich innerhalb des Tumors erreicht werden oder es können Bereiche außerhalb des Tumors erreicht werden, um in Entstehung begriffene Metastasen des Tumors, die z.B. bilddiagnostisch noch nicht voll erfasst werden können, zu zerstören.

## Patentansprüche

1. Planungsverfahren zum Planen mindestens eines Infusions- und/oder Injektionsbereiches (A5) zur Injektion eines medizinischen Wirkstoffes in eine anisotrope Körperstruktur (10), die biologisches Gewebe enthält, um eine Verteilung des medizinischen Wirkstoffes in einem gewünschten Zielbereich (A1) der anisotropen Körperstruktur (10) zu erzielen, mit folgenden Schritten:
mindestens eine Eigenschaft der anisotropen Körperstruktur (10), nämlich die kapillare Permeabilität oder hydraulische Leitfähigkeit der anisotropen Körperstruktur (10), die die räumliche Verteilung des medizinischen Wirkstoffes in der anisotropen Körperstruktur (10) beeinflusst, wird mittels Magnetresonanztomographie (MRT) und/oder Magnetresonanzangiographie (MRA) und/oder funktioneller Magnetresonanztomographie (fMRI) und/oder Bildgebung des zellularen Blutflusses (ASL) und/oder Diffusionsbildgebung (DWI) und/oder Perfusionsbildgebung (PWI) und/oder MR-Spektroskopie (MRS, SVS, CSI) und/oder Diffusions-Tensor-Bildgebung (DTI) bereitgestellt;
**dadurch gekennzeichnet, dass**
der mindestens eine Infusions- und/oder Injektionsbereich (A5), von dem ausgehend ein Transport des medizinischen Wirkstoffes durch die anisotrope Körperstruktur zu mindestens einem Zielbereich (A1) erfolgen soll, wird zumindest in Abhängigkeit von der mindestens einen Eigenschaft der anisotropen Körperstruktur, nämlich der kapillaren Permeabilität oder hydraulischen Leitfähigkeit der anisotropen Körperstruktur (10), und einer Lage des gewünschten mindestens einen Zielbereichs (A1) mittels eines Computers bestimmt, wobei die anisotrope Körperstruktur räumlich in Segmente aufgeteilt wird und die Wahrscheinlichkeit des Wirkstofftransports von einem dieser Segmente zu einem weiteren dieser Segmente bestimmt wird.

2. Verfahren nach Anspruch 1, bei welchem basierend auf der mindestens einen Eigenschaft ein Wirkstoffverlust in Segmenten (A1-C5) der anisotropen Körperstruktur bestimmt wird und basierend auf dem für die Segmente bestimmten Wirkstoffverlust der mindestens eine Injektionsbereich bestimmt wird.

3. Verfahren nach Anspruch 1 oder 2, bei welchem die Verbundenheit und/oder der Wirkstoffverlust durch die Permeabilität und/oder hydraulischen Leitfähigkeitseigenschaften der Segmente bestimmt wird.

4. Verfahren nach einem der vorhergehenden Ansprüche, bei welchem die Verbundenheit und/oder der Wirkstoffverlust durch die Anordnung und/oder Größe und/oder Gestalt der Segmente, und/oder Anzahl der angrenzenden Segmente bestimmt wird.

5. Verfahren nach einem der vorhergehenden Ansprüche, bei welchem eine Wahrscheinlichkeit für die Verbundenheit und/oder den Wirkstoffverlust berechnet wird, und hieraus eine Wahrscheinlichkeit für möglicherweise für eine Injektion geeignete Segmente berechnet wird, mit welcher bei Injektion eine Wirkstoffverteilung in dem mindestens einem Zielbereich erzielt werden kann.

6. Verfahren nach einem der vorhergehenden Ansprüche, bei welchem zumindest eines der folgenden Bestimmungsergebnisse visualisiert wird:
ein oder mehrere mögliche Wirkstofftransportpfade vom bestimmten mindestens einem Infusion und/oder Injektionsbereich zum mindestens einem Zielbereich;
Wahrscheinlichkeit für Segmente der anisotropen Körperstruktur, dass bei Infusions- und/oder Injektion des Wirkstoffes in die Segmente eine Verteilung des medizinischen Wirkstoffes im Zielbereich erzielt wird;
die Verbundenheit benachbarter Segmente;
den Wirkstoffverlust in Segmenten;
mögliche zur Injektion geeignete Segmente der anisotropen Körperstruktur.

7. Verfahren nach einem der vorhergehenden Ansprüche, bei welchem ein zu erwartendes Verhältnis der Konzentration und/oder Menge des zur Injektion vorgesehenen medizinischen Wirkstoffes in dem mindestens einem Zielbereich zu der injizierten Konzentration und/oder Menge zumindest in Abhängigkeit von der mindestens einen Eigenschaft der anisotropen Körperstruktur und der berechneten Lage des mindestens einen Injektionsbereich berechnet wird.

8. Verfahren nach Anspruch 7, bei welchem das zu erwartende Verhältnis und/oder die Konzentration und/oder die Menge des zur Injektion vorgesehenen medizinischen Wirkstoffes zumindest in Abhängigkeit mindestens einer Wirkstoffeigenschaft und/oder mindestens einer Injektionseigenschaft berechnet wird.

9. Verfahren nach einem der vorhergehenden Ansprüche, bei welchem die zu erwartende, von den Zellen im Wirkbereich insgesamt oder pro Zeiteinheit absorbierte Menge des medizinischen Wirkstoffes, basierend auf der für den Zielbereich berechneten Konzentration und auf den Wirkstoff-Absorptionseigenschaften der Zellen im Wirkbereich berechnet wird.

10. Verfahren nach einem der vorhergehenden Ansprüche,
bei welchem die Eigenschaft der anisotropen Körperstruktur zumindest eine der folgenden Eigenschaften umfasst:
Eigenschaften, die den Transport des medizinischen Wirkstoffes, insbesondere von mittels Flüssigkeit transportfähigen Wirkstoff-Molekülen, in der anisotropen Körperstruktur, insbesondere auf Grund von Körperflüssigkeit-Transportvorgängen beeinflussen;
Verlauf und Lage von Senken für den medizinischen Wirkstoff in der anisotropen Körperstruktur;
Eigenschaften, die die Absorption des medizinischen Wirkstoffes in der anisotropen Körperstruktur beeinflussen;
Eigenschaften, die die Diffusion des medizinischen Wirkstoffes im Wirkbereich beeinflussen;
die hydraulische Leitfähigkeit von in der anisotropen Körperstruktur verlaufenden, zum Wirkstofftransport geeigneten Teilen der anisotropen Körperstruktur, wie von Gefäßen und/oder Nervensträngen und/oder Sulci und/oder Hohlräumen und/oder Zellen insbesondere zu benachbarten Teilen, wobei sich die benachbarten Teile in ihrer biologischen Eigenschaft unterscheiden können;
die Transportleistung für den Wirkstoff von Teilen der anisotropen Körperstruktur, insbesondere von Gefäßen und/oder Nervensträngen und/oder Sulci und/oder Hohlräumen;
der Wirkstoffverlust durch sich in ihrer biologischen Eigenschaft unterscheidende Teile der anisotropen Körperstruktur, insbesondere durch Zellen und/oder Gefäße und/oder Nervenstränge und/oder Sulci und/oder Hohlräume;
Perkolationseigenschaften der anisotropen Körperstruktur; und
vorgesehene Lage des Patienten.

11. Planungsverfahren zum Planen einer Infusion und/oder Injektion eines medizinischen Wirkstoffes in eine anisotrope Körperstruktur, wobei die gewünschte Lage oder die gewünschten Lagen des mindestens einen Zielbereichs vorgegeben wird bzw. werden und basierend auf dem Verfahren nach einem der Ansprüche 1 bis 10 der mindestens eine Infusions- und/oder Injektionsbereich berechnet wird.

12. Verfahren nach einem der Ansprüche 1 bis 11, das auf einem Computer ausgeführt wird.

13. Vorrichtung zum Bestimmen mindestens eines Infusions- und/oder Injektionsbereiches (A5) zur Infusion und/oder Injektion eines medizinischen Wirkstoffes in eine anisotrope Körperstruktur (10), die biologisches Gewebe enthält, um eine Verteilung des medizinischen Wirkstoffes in einem gewünschten Zielbereich (A1) der anisotropen Körperstruktur (10) zu erzielen, umfassend:
ein Bereitstellungsmittel, das mindestens eine Eigenschaft der anisotropen Körperstruktur (10), nämlich die kapillare Permeabilität oder hydraulische Leitfähigkeit der heterogenen Körperstruktur (10), die die räumliche Verteilung des medizinischen Wirkstoffes in der anisotropen Körperstruktur (10), beeinflusst, mittels Magnetresonanztomographie (MRT) und/oder Magnetresonanzangiographie (MRA) und/oder funktionelle Magnetresonanztomographie (fMRI) und/oder Bildgebung des zellularen Blutflusses (ASL) und/oder Diffusionsbildgebung (DWI) und/oder Perfusionsbildgebung (PWI) und/oder MR-Spektroskopie (MRS, SVS, CSI) und/oder Diffusions-Tensor-Bildgebung (DTI) bereitgestellt;
eine Eingabemittel zum Eingeben der gewünschten Lage mindestens eines Wirkbereichs;
ein Berechnungsmittel,
**dadurch gekennzeichnet, dass**
das Berechnungsmittel einen Computer umfasst, der ausgebildet ist,
den mindestens einen Injektionsbereich (A5), von dem ausgehend ein Transport des medizinischen Wirkstoffes durch die anisotrope Körperstruktur zu dem mindestens einem Zielbereich erfolgen soll, zumindest in Abhängigkeit von der mindestens einen Eigenschaft der anisotropen Körperstruktur, nämlich der kapillaren Permeabilität oder hydraulischen Leitfähigkeit der anisotropen Körperstruktur (10), und der Lage des gewünschten mindestens einen Zielbereichs (A1) zu berechnen, wobei die anisotrope Körperstruktur räumlich in Segmente aufgeteilt wird und die Wahrscheinlichkeit des Wirkstofftransports von einem dieser Segmente zu einem weiteren dieser Segmente bestimmt wird.

14. Vorrichtung nach Anspruch 13, weiter umfassend:
ein Injektionsinstrument zum Injizieren des medizinischen Wirkstoffes; und/oder
eine Überwachungseinheit zum Bestimmen der Lage des Infusions- und/oder Injektionsinstrumentes in der anisotropen Körperstruktur; und/oder
eine Diagnoseeinheit zum Bestimmen der mindestens einen Eigenschaft der anisotropen Körperstruktur und zum Erzeugen von die mindestens eine Eigenschaft beschreibenden Daten; und/oder
eine Datenbank, die Daten über den Wirkstofftransport und/oder Wirkstoffverlust in Teilen oder zwischen Teilen der anisotropen Körperstruktur, die sich insbesondere in ihren biologischen Eigenschaften unterscheiden, wie z.B. von Zellen und/oder Gefäßen und/oder Nervensträngen und/oder Sulci und/oder Hohlräumen gespeichert hat, wobei das Berechnungsmittel ausgebildet ist, auf die Daten der Diagnoseeinheit und/oder der Datenbank zuzugreifen, um die Berechnung durchzuführen.

## Claims

1. A planning method for planning at least one infusion and/or injection region (A5) for injection of an active medical substance into an anisotropic body structure (10) which contains biological tissue in order to achieve a distribution of the active medical substance in a desired target region (A1) of the anisotropic body structure (10), comprising the following steps:
at least one property of the anisotropic body structure (10) namely the capillary permeability or the hydraulic conductivity of the anisotropic body structure (10), which influences the spatial distribution of the active medical substance in the anisotropic body structure (10) is provided by magnetic resonance tomography (MRT) and/or magnetic resonance angiography (MRA) and/or functional magnetic resonance tomography (fMRI) and/or imaging the cellular blood flow (ASL) and/or diffusion imaging (DWI) and/or perfusion imaging (PWI) and/or MR spectroscopy (MRS, SVS, CSI) and/or diffusion tensor imaging (DTI);
**characterized in that**
the at least one infusion and/or injection area (A5), outgoing from which a transport of the active medical substance through the anisotropic body structure to at least one target region (A1) shall occur, is determined at least in dependence on the at least one property of the anisotropic body structure, namely the capillary permeability or hydraulic conductivity of the anisotropic body structure (10), and a position of the desired at least one target region (A1) by using a computer, wherein the anisotropic body structure is spatially divided into segments and the probability of transport of the active substance from one of these segments to a further one of these segments is determined.

2. The method according to claim 1, wherein a loss of the active substance in segments (A1-C5) of the anisotropic body structure is determined based on the at least one property and the at least one injection region is determined based on the loss of active substance determined for the segments.

3. The method according to claim 1 or 2, wherein the connectivity and/or the loss of active substance is determined through the permeability and/or hydraulic conductivity properties of the segments.

4. The method according to anyone of the preceding claims, wherein the conductivity and/or the loss of active substance is determined through the arrangement and/or size and/or shape of the segments and/or the number of the adjacent segments.

5. The method according to anyone of the preceding claims, wherein a probability for the connectivity and/or the loss of active substance is calculated and, based on this, the probability for segments which may be suitable for an injection is calculated, using which probability a distribution of the active substance in the at least one target region can be achieved if injection is performed.

6. The method according to anyone of the preceding claims, wherein at least one of the following determination results is visualized:
one or more possible transport paths for the active substance from the determined at least one infusion and/or injection region to the at least one target region;
a probability for segments of the anisotropic body structure that, in case of infusion and/or injection of the active substance into the segments, a distribution of the active medical substance in the target region is achieved;
the connectivity of adjacent segments;
the loss of substance in segments;
possible segments of the anisotropic body structure which are suitable for injection.

7. The method according to anyone of the preceding claims, wherein an inspected ratio of the concentration and/or amount of the active medical substance intended for injection in the at least one target region to the injected concentration and/or amount is calculated at least in dependence on the at least one property of the anisotropic body structure and the calculated position of the at least one injection region.

8. The method according to claim 9, wherein the expected ratio and/or concentration and/or amount of the active medical substance intended for injection is calculated at least in dependence on one of the substance property and/or at least one injection property.

9. The method according to anyone of the preceding claims, wherein the expected amount of the active medical substance absorbed by the cells in the effect region in total or per time unit is calculated based on the concentration calculate for the target region and the absorption properties of the cells in the effect region for the active substance.

10. The method according to anyone of the preceding claims,
wherein the property of the anisotropic body structure comprises at least one of the following properties:
properties which influence the transport of the active medical substance in the anisotropic body structure in particular by active substance molecules transportable in fluids and in particular due to transport processes of body fluids;
the run and position of drains for the active medical substance in the anisotropic body structure;
properties which influence the absorption of the active medical substance in the anisotropic body structure;
properties which influence the diffusion of the active medical substance in the effect region;
the hydraulic conductivity of parts of the anisotropic body structure running in the anisotropic body structure and suitable for transport of the active substance, for example vessels and/or nerve fibres and/or sulci and/or cavities and/or cells in particular towards neighbouring parts, wherein the neighbouring parts may differ in their biologic property;
the transport efficiency for the active substance of parts of the anisotropic body structure in particular of vessels and/or nerve fibres and/or sulci and/or cavities;
the loss of active substance due to parts of the anisotropic body structure differing in their biologic property, in particular due to cells and/or vessels and/or nerve fibres and/or sulci and/or cavities;
percolation properties of the anisotropic body structure; and
the intended position of the patient.

11. The planning method according to anyone of claims 1 to 12 having an infusion and/or injection of an active medical substance into an anisotropic body structure, wherein the desired position and/or the desired positions of the at least one target region is or are predetermined and the at least one infusion and/or injection region is calculated based on the method according to anyone of claims 1 to 12.

12. The method according to anyone of claims 1 to 11, wherein the method is executed on a computer.

13. An apparatus for determining at least one infusion and/or injection region (A5) for infusion and/or injection of an active medical substance into an anisotropic body structure (10) containing biologic tissue in order to achieve a distribution of the active medical substance in a desires target region (A1) of the anatomical body structure (10), the apparatus comprising:
providing means which provide at least one property of the anisotropic body structure (10), namely the capillarity permeability or hydraulic conductivity of the anisotropic body structure (10), which influences the spatial distribution of the active medical substance in the anisotropic body structure (10) by magnetic resonance tomography (MRT) and/or magnetic resonance angiography (MRA) and/or functional magnetic resonance tomography (fMRI) and/or imaging the cellular blood flow (ASL) and/or diffusion imaging (DWI) and/or perfusion imaging (PWI) and/or MR spectroscopy (MRS, SVS, CSI) and/or diffusion tensor imaging (DTI);
inputting means for inputting the desired position of at least one effect region;
computing means,
**characterized in that**
the computing means comprise a computer which is adapted to
calculate the at least one injection region (A5), outgoing from which a transport of the active medical substance through the isotropic body structure to the at least one target region shall occur, at least in dependence on the at least one property of the anisotropic body structure, namely the capillary permeability or hydraulic conductivity of the anisotropic body structure (10), and the position of the desired at least one target region (A1), wherein the anisotropic body structure is divided spatially into segments and the probability of transport of the active substance from one of these segments to a further one of these segments is determined.

14. The apparatus according to claim 13, further comprising:
an injection instrument for injecting the active medical substance and/or
a surveillance unit for determining the position of the infusion and/or injection instrument in the anisotropic body structure; and/or
a diagnosis unit for determining the at least one property of the anisotropic body structure and for generating data describing the at least one property; and/or
a database storage data about the transport of the active substance and/or loss of the active substance in parts or between parts of the anisotropic body structure differing in particular in their biologic properties such as cells and/or vessels and/or nerve fibres and/or sulci and/or cavity, wherein the calculating means is adapted to access the data of the diagnosis unit and/or database in order to perform the calculation.

## Revendications

1. Procédé de planification pour la planification d'au moins une zone de perfusion et/ou d'injection (A5) pour l'injection d'un principe actif médical dans une structure corporelle anisotrope (10) contenant un tissu biologique, afin d'obtenir une distribution du principe actif médical dans une zone cible (A1) désirée de la structure corporelle anisotrope (10), comportant les étapes suivantes :
au moins une propriété de la structure corporelle anisotrope (10), à savoir la perméabilité capillaire ou la conductivité hydraulique de la structure corporelle anisotrope (10) qui influence la distribution spatiale du principe actif médical dans la structure corporelle anisotrope (10) est mise à disposition en utilisant l'imagerie par résonance magnétique (IRM) et/ou l'angiographie par résonance magnétique (ARM) et/ou l'imagerie par résonance magnétique fonctionnelle (IRMf) et/ou l'imagerie par marquage de spin artériels (ASL) et/ou l'imagerie pondérée en diffusion (DWI) et/ou l'imagerie pondérée en perfusion (PWI) et/ou la spectroscopie par résonance magnétique (MRS, SVS, CSI) et/ou l'imagerie du tenseur de diffusion (DTI) ;
**caractérisé en ce que**
l'au moins une zone de perfusion et/ou d'injection (A5) à partir de laquelle doit s'effectuer un transport du principe actif médical à travers la structure corporelle anisotrope jusqu'à au moins une zone cible (A1) est déterminée au moyen d'un ordinateur au moins en fonction de l'au moins une propriété de la structure corporelle anisotrope, à savoir la perméabilité capillaire ou la conductivité hydraulique de la structure corporelle anisotrope (10) et d'une position de l'au moins une zone cible (A1) désirée, où la structure corporelle anisotrope est divisée spatialement en segments et la probabilité d'un transport du principe actif depuis l'un de ces segments vers un autre de ces segments est déterminée.

2. Procédé selon la revendication 1, dans lequel une perte de principe actif dans des segments (A1-C5) de la structure corporelle anisotrope est déterminée en se fondant sur l'au moins une propriété et l'au moins une zone d'injection est déterminée en se fondant sur la perte de principe actif déterminée dans les segments.

3. Procédé selon la revendication 1 ou 2, dans lequel le degré de liaison et/ou la perte de principe actif est déterminé par la perméabilité et/ou les propriétés de conductivité hydraulique.

4. Procédé selon l'une quelconque des revendications précédentes, dans lequel le degré de liaison et/ou la perte de principe actif est déterminé par l'agencement et/ou la taille et/ou la forme des segments, et/ou le nombre de segments adjacents.

5. Procédé selon l'une quelconque des revendications précédentes, dans lequel une probabilité du degré de liaison et/ou de la perte de principe actif est calculée et une probabilité de pouvoir obtenir une distribution du principe actif dans l'au moins une zone cible lors d'une injection est calculée pour des segments potentiellement adaptés à une injection.

6. Procédé selon l'une quelconque des revendications précédentes, dans lequel au moins un des résultats de détermination suivants est visualisé :
un ou plusieurs chemins de transport du principe actif possibles entre l'au moins une zone de perfusion et/ou d'injection déterminée et l'au moins une zone cible ;
la probabilité, pour des segments de la structure corporelle anisotrope, d'obtenir une distribution du principe actif médical dans la zone cible lors d'une perfusion et/ou injection du principe actif dans les segments ;
le degré de liaison entre des segments adjacents ;
la perte de principe actif dans des segments ;
des segments de la structure corporelle anisotrope adaptés pour une injection.

7. Procédé selon l'une quelconque des revendications précédentes, dans lequel un rapport attendu entre la concentration et/ou quantité du principe actif médical destiné à être injecté dans l'au moins une zone cible et la concentration et/ou quantité injectée dans l'au moins une zone cible est calculée au moins en fonction de l'au moins une propriété de la structure corporelle anisotrope et de la position calculée de l'au moins une zone d'injection.

8. Procédé selon la revendication 9, dans lequel le rapport attendu et/ou la concentration et/ou la quantité du principe actif médical destiné à être injecté est calculé au moins en fonction d'au moins une propriété du principe actif et/ou au moins une propriété de l'injection.

9. Procédé selon l'une quelconque des revendications précédentes, dans lequel la quantité attendue totale ou par unité de temps de principe actif médical absorbée par les cellules se trouvant dans la zone d'action est calculée en se fondant sur la concentration et sur les propriétés d'absorption du principe actif des cellules se trouvant dans la zone d'action.

10. Procédé selon l'une quelconque des revendications précédentes,
dans lequel la propriété de la structure corporelle anisotrope comprend au moins une des propriétés suivantes :
des propriétés qui influencent le transport du principe actif médical dans la structure corporelle anisotrope, en particulier de molécules du principe actif aptes à être transportées au moyen d'un fluide, en particulier sur la base de processus de transport par des fluides corporels ;
le tracé et la position de creux dans la structure corporelle anisotrope pour le principe actif médical ;
des propriétés qui influencent l'absorption du principe actif médical dans la structure corporelle anisotrope ;
des propriétés qui influencent la diffusion du principe actif médical dans la zone d'action ;
la conductivité hydraulique de parties de la structure corporelle anisotrope, comme par exemple de vaisseaux et/ou fibres nerveuses et/ou de sulci et/ou de cavités et/ou de cellules, s'étendant dans la structure corporelle anisotrope et adaptées au transport du principe actif, en particulier par rapport à des parties adjacentes, où les parties adjacentes peuvent être différentes en termes de leur propriété biologique ;
la capacité de transport du principe actif de certaines parties de la structure corporelle anisotrope, en particulier de vaisseaux et/ou de fibres nerveuses et/ou de sulci et/ou de cavités ;
la perte de principe actif par des parties de la structure corporelle anisotrope différentes en termes de leur propriété biologique, en particulier par des cellules et/ou des vaisseaux et/ou des fibres nerveuses et/ou des sulci et/ou des cavités ;
des propriétés de percolation de la structure corporelle anisotrope ; et
la position prévue du patient.

11. Procédé de planification pour la planification d'une perfusion et/ou d'une injection d'un principe actif médical dans une structure corporelle anisotrope, où la position ou les positions désirées de l'au moins une zone cible est ou sont données et l'au moins une zone de perfusion et/ou d'injection est calculée en se fondant sur le procédé selon l'une quelconque des revendications 1 à 12.

12. Procédé selon l'une quelconque des revendications 1 à 11, qui peut être exécuté par un ordinateur.

13. Dispositif pour déterminer au moins une zone de perfusion et/ou d'injection (A5) pour la perfusion et/ou l'injection d'un principe actif médical dans une structure corporelle anisotrope (10) contenant un tissu biologique, afin d'obtenir une distribution du principe actif médical dans une zone cible (A1) désirée de la structure corporelle anisotrope (10), comportant :
un moyen de mise à disposition qui met à disposition au moins une propriété de la structure corporelle anisotrope (10) qui influence la distribution spatiale du principe actif médical dans la structure corporelle anisotrope (10), à savoir la perméabilité capillaire ou la conductivité hydraulique de la structure corporelle hétérogène (10), en utilisant l'imagerie par résonance magnétique (IRM) et/ou l'angiographie par résonance magnétique (ARM) et/ou l'imagerie par résonance magnétique fonctionnelle (IRMf) et/ou l'imagerie par marquage de spin artériels (ASL) et/ou l'imagerie pondérée en diffusion (DWI) et/ou l'imagerie pondérée en perfusion (PWI) et/ou la spectroscopie par résonance magnétique (MRS, SVS, CSI) et/ou l'imagerie du tenseur de diffusion (DTI) ;
un moyen de saisie pour saisir la position désirée d'au moins une zone d'action ;
un moyen de calcul,
**caractérisé en ce que**
le moyen de calcul comporte un ordinateur qui est configuré pour
calculer l'au moins une zone d'injection (A5) à partir de laquelle doit s'effectuer un transport du principe actif médical à travers la structure corporelle anisotrope jusqu'à l'au moins une zone cible (A1), au moins en fonction de l'au moins une propriété de la structure corporelle anisotrope, à savoir la perméabilité capillaire ou la conductivité hydraulique de la structure corporelle anisotrope (10) et de la position de l'au moins une zone cible (A1) désirée, où la structure corporelle anisotrope est divisée spatialement en segments et la probabilité d'un transport du principe actif depuis l'un de ces segments vers un autre de ces segments est déterminée.

14. Dispositif selon la revendication 13, comprenant en outre :
un instrument d'injection pour injecter le principe actif médical ; et/ou
une unité de surveillance pour déterminer la position de l'instrument de perfusion et/ou d'injection dans la structure corporelle anisotrope ; et/ou
une unité de diagnostic pour déterminer l'au moins une propriété de la structure corporelle anisotrope et pour générer des données décrivant l'au moins une propriété ; et/ou
une base de données dans laquelle sont stockées des données concernant le transport de principe actif et/ou la perte de principe actif dans des parties ou entre des parties de la structure corporelle anisotrope, comme par exemple des cellules et/ou des vaisseaux et/ou des fibres nerveuses et/ou des sulci et/ou des cavités qui se distinguent en particulier en termes de leurs propriétés biologiques, où l'élément de calcul est configuré pour accéder aux données de l'unité de diagnostic et/ou de la base de données, pour effectuer le calcul.
